(19) **Europäisches Patentamt**
**European Patent Office**
**Office européen des brevets**

(11) **EP 4 007 917 B1**

(12) **EUROPEAN PATENT SPECIFICATION**

(45) Date of publication and mention
of the grant of the patent:
**11.10.2023 Bulletin 2023/41**

(21) Application number: **20734529.9**

(22) Date of filing: **23.06.2020**

(51) International Patent Classification (IPC):
***A61M 1/36*** *(2006.01)*     ***A61M 1/34*** *(2006.01)*
*G01N 33/49* *(2006.01)*

(52) Cooperative Patent Classification (CPC):
**A61M 1/3496; A61M 1/3672;** A61M 1/342;
A61M 2230/207; G01N 33/491

(86) International application number:
**PCT/EP2020/067529**

(87) International publication number:
**WO 2021/018481 (04.02.2021 Gazette 2021/05)**

(54) **METHOD FOR CONTROLLING THE AMOUNT OF ANTICOAGULANT PRESENT IN COLLECTED PLASMA AFTER APHERESIS**

VERFAHREN ZUR KONTROLLE DER MENGE AN GERINNUNGSHEMMENDEM MITTEL IM GESAMMELTEN PLASMA NACH DER APHERESE

PROCÉDÉ DE RÉGULATION DE LA QUANTITÉ D'ANTICOAGULANT PRÉSENT DANS UN PLASMA COLLECTÉ APRÈS APHÉRÈSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.08.2019 US 201962881781 P**

(43) Date of publication of application:
**08.06.2022 Bulletin 2022/23**

(73) Proprietor: **Grifols Worldwide Operations Limited Dublin 22 (IE)**

(72) Inventor: **FLEXMAN, Greg**
**WEST CLAYTON, North Carolina 27520 (US)**

(74) Representative: **Durán-Corretjer, S.L.P.**
**Còrsega, 329**
**(Paseo de Gracia/Diagonal)**
**08037 Barcelona (ES)**

(56) References cited:
**EP-A1- 2 896 415      WO-A1-2019/085156**
**JP-A- 2012 081 213**

**Description**

The present invention relates to the field of methods for collecting blood components. in particular, it refers to a method for controlling the amount of anticoagulant in plasma collected after apheresis.

Apheresis is a procedure in which individual blood components are collected and separated from whole blood that is temporarily withdrawn from a donor. Typically, whole blood is withdrawn through a needle inserted into a vein of a donor's arm; anticoagulant is added to the blood, and the mixture is pumped into a separator such as a centrifugal bowl. Whole blood will coagulate or "clot" shortly after removal from a donor unless an anticoagulant is added. Typical anti-coagulants include calcium chelators such as ethylenedi-aminetetraacetic acid (EDTA), oxalate (oxalic acid) or citrate (citric acid). These anticoagulants function by chelating calcium ions in the blood that impedes clot formation (thrombosis). The most common anticoagulant is sodium citrate and/or citric acid suspended in a dextrose (glucose) solution.

Once the whole blood is separated into its various components based on physical characteristics such as density (e.g., red blood cells (RBC), white blood cells, platelets, and plasma), one or more of the components can be removed from the separation device. The remaining components can be returned to the donor, in some cases with optional compensation fluid to make up for the volume of the removed component or to facilitate transit. The compensation fluid is often saline. The process of drawing whole blood and returning the separated components to the donor continues until the quantity of the desired component has been collected, at which point the process is stopped. A central feature of apheresis systems is that the processed but unwanted components are returned to the donor. Separated blood components may include, for example, a high-density component such as RBC, an intermediate-density component such as white blood cells or platelets, or a low density component such as plasma.

Apheresis systems are used widely for the collection of single donor platelets and single donor plasma. A central feature of apheresis devices, however, is that, while they separate blood components at the point of collection, the unwanted blood components are returned to the donor. Accordingly, apheresis devices must incorporate a variety of safety features, such as air detectors and pressure monitors, to protect the donor from harm while blood components are collected and separated. Such safety mechanisms add complexity to apheresis system equipment but are essential for the safety and protection of the donor. Protections include measures to ensure that only desired components are returned to the donor (such as RBC), and potentially harmful substances such as pure anticoagulant or air are not introduced to the donor under any circumstances. Collection methods must also ensure that all blood drawn from a donor must be mixed with anticoagulant, to ensure that uncontrolled coagulation does not commence, possibly affecting the donor upon return of components, and possibly affecting the quality of the collected components.

A basic automated blood collection device used to carry out apheresis typically consist of: a reservoir holding anticoagulant solution; a pump for pumping the anticoagulant solution; a needle (or other cannula-like device) for insertion into a vein of the donor and drawing whole blood; a mixing means for mixing anticoagulant with whole blood (e.g., a Y-connector); a pump to propel anticoagulant to be mixed with whole blood; a pump for pumping anticoagulated blood to the separator and returning select components to the donor; separator means; and a reservoir for collecting the desired blood component; tubing for various interconnections; liquid/air sensors or detectors for determining the location of fluids within the tubes; and pressure sensors for determining the pressure of the fluid within the tubes or lines. Specific examples of automated blood collection devices useful with the methods described herein are described in U.S. Pat. Nos. 5,387,187; 5,494,592; and 5,637,082 and WO 2009/129140.

Other useful collection devices would be recognized by one having ordinary skill in the art. Plasmapheresis donors presenting for plasma collection usually undergo a series of screening processes that measure their current physical condition and determine eligibility to donate. This includes measures of weight and HCT (hematocrit). HCT, also known by several other names, is a blood test that measures the volume percentage (vol%) of RBC in blood. The measurement depends on the number and size of RBC. it is normally 40.7% to 50.3% for men and 36.1% to 44.3% for women.

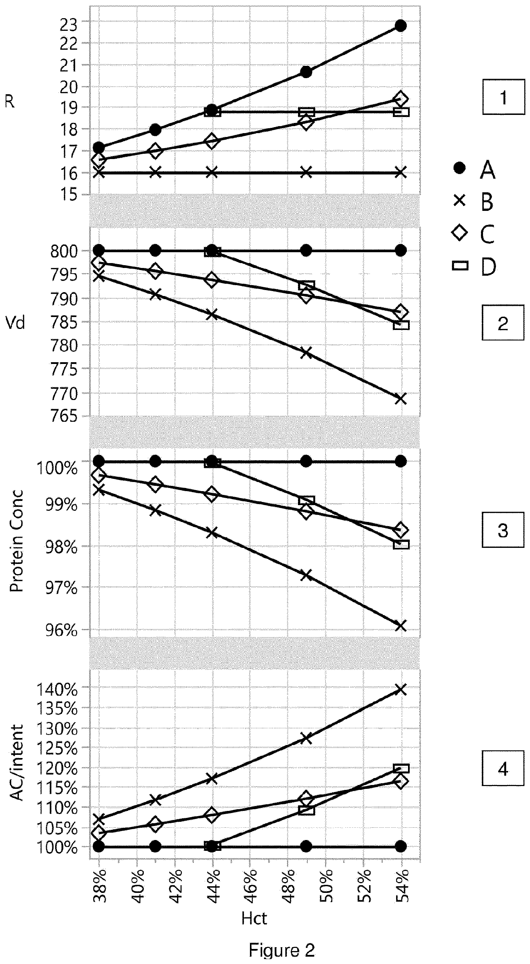

A variety of strategies have been presented by suppliers of collection equipment to manage the flow of whole blood, the introduction of anticoagulant (AC) and the separation into collected anticoagulated plasma, and return of RBC to the donor. Blood processing systems, such as apheresis systems, typically add an anticoagulant (e.g., sodium citrate/citric acid in saline or dextrose solution) to prevent blood or blood components from clumping and coagulating during collection within the blood collection system or in the collected components. Typically, the anticoagulant is directly mixed with the whole blood as it is drawn from the donor. The ratio of anticoagulant to whole blood may be set to a specific ratio. For example, the PCT application WO1994/012223A1 discloses that for plasma collection, the ratio of AC to anticoagulated blood may be 1:16 (i.e., 0.06; one part 4% sodium citrate anticoagulant for every 16 parts of anticoagulated blood). This fixed ratio, without regard to HCT, is illustrated on Figure 2(1), line B.

However, the U.S. Food and Drug Administration (FDA) has published expectations for volumes to target when collecting plasma. See, e.g., Kathryn C. Zoon, Director, FDA Center for Biologics Evaluation and Research, Volume Limits for Automated Collection of Source Plasma (Nov. 4, 1992). A FDA issued nomogram "Volume limits- automated collection of Source Plasma" currently forms the basis for all US plasma collections. This FDA document describes limits for two elements: (1) plasma volume (the plasma that is removed from the donor), and (2) collected volume (the net amount of plasma + AC that will be used for manufacturing). These limits are described as three pairs of values, to be applied according to three donor weight categories. The "intended AC" in the collected product can be inferred as the difference between the collected amount, and plasma volume. it is notable that this is a constant 9% proportion across donor weight groups.

However, when a 1:16 ratio is used as a ratio of volumetric flow between the propulsion of AC towards the mixing point, and the draw of anticoagulated (mixed) whole blood, the relationship between plasma volume and collected volume results with an excess of AC present in the collected volume, as compared to the intended FDA inferred amount. The amount of excess AC is variable, and increases along with the HCT of the patient.

Hematocrit-based estimation of an amount of anticoagulant in apheresis is known from WO 2019/085156 A1, JP 2012 081213 A and EP 2 896 415 A1.

In practice, the above-mentioned drawback has been not been previously solved in commercially available systems. Some existing control systems using a fixed 1:16 ratio target the termination of the process at the collected volume, in which case the excess AC present in the collected volume means that the donation is short of the allowed amount of plasma volume from donor. When the 1:16 ratio is used, donors with lower range HCT values were nearly donating the recommended amount in plasma, whereas donors with typical, or higher HCT values were stopped short from completing the full donation amount as the collected volume would be reached by the excess of AC needed with the extra whole blood flow to mechanically separate the RBC from the plasma. This reduction in the volume from donor (Vd) according to HCT is illustrated in Figure 2(2), line B status quo for an example where 800 mL is intended to be withdrawn from the donor.

Other existing manifestations also use a fixed 1:16 ratio, but use knowledge of the HCT of that particular donor to infer the excess AC in the collected volume, and continue the collection until the targeted amount of plasma from donor has been withdrawn. This results in larger amounts of collected volume. While this does draw the allowed amount of plasma from the donor, the excess volume can be physically challenging to extract from the bottle. There is also an excess of AC present in the collected material.

Therefore, the excess AC can cause issues such as: ceasing collection before donor amount target reached, or increasing the volume in the collection bottle which is physically challenging to remove larger volumes from bottle, and in all cases of fixed AC ratio dilution of the plasma intended to be sent to the fractionation process thereby reducing process step throughput capacity. This dilution relative to the intended amount of plasma from donor in the is illustrated in Figure 2(3) line B for an example where 800 mL is intended to be withdrawn from the donor and blended with 80 mL of AC for a collection volume of 880 mL. Higher amounts of AC than needed may also adversely affect proteins, which could reduce process yield of usable material. The excess portion of AC from a fixed ratio collection, relative to the FDA nomogram intended amount (e.g. 80mL in an 880 collection), is illustrated in Figure 2(4) line B.

in order to overcome the above-mentioned drawbacks, the inventors of the present invention have discovered that using a variable AC to anticoagulated blood ratio, depending on the particular HCT of the donor undergoing the process, it is possible to control the amount of AC in the collected plasma to a desired intended amount, and reduce the degree of unintended influence of HCT, allowing that the amount of AC in the collected plasma is lower than the value recommended by the FDA. This HCT value is routinely measured prior to donation as part of donor intake process, and can be transferred to the collection equipment either manually or electronically, and/or derived from observation of component flows on the equipment during collection. in this manner, it is possible to target both FDA nomogram parameters of donated plasma and collected volume diminishing the excess AC present on normal or higher HCT collections. The AC pump could be controlled at an alternate (variable) ratio to the anticoagulated blood pump, and/or incorporate the time rate of change observed in the collected volume in the optimal targeted AC flow calculation.

Therefore, in a first aspect, the present invention discloses a method for controlling the concentration of an anticoagulant composition added to a donor's plasma during a fixed volume apheresis extraction process, the method comprising utilising a donor's hematocrit (HCT) measurement to determine the ratio of the anticoagulant composition to the donor's uncoagulated blood during the apheresis extraction process.

In the method of the present invention said ratio of the anticoagulant composition to the donor's uncoagulated blood during the apheresis extraction process is determined as a function of at least the donor's HCT measurement, and:

    i) The total volume of collected plasma ($V_C$); and
    ii) The desired volume of the anticoagulant in the collected plasma ($V_{AC}$).

In the method of the present invention said ratio of the anticoagulant composition to the donor's uncoagulated blood during the apheresis extraction process is 1:R, wherein R is calculated according to the following equation:

$$R = \frac{{}^{V_C}\!/_{V_{AC}} - HCT}{1 - HCT}$$

Wherein $V_C$ is the total volume of collected plasma (mL); $V_{AC}$ is the volume of anticoagulant in the collected plasma (mL), and HCT is the patient's hematocrit expressed in decimals; i.e. a value of 44% of HCT should be used in the above formula as 0.44.

For example, when the total volume of collected plasma ($V_C$) is 880 mL, and the desired content of anticoagulant ($V_{AC}$) is 80 mL, and the patient's HCT is 0.44, the resulting ratio (R) is 18.86. To operate in this manner, the AC pump (2) would propel 1 unit of flow for every 18.857 units of flow through pump (11).

in another aspect, the present invention discloses a method for controlling the amount of AC present in collected plasma from a donor after apheresis, comprising the steps of:

a) measuring the HCT value in said donor's blood;
b) calculating the AC to anticoagulated blood ratio in view of said HCT value of step a);
c) carrying out the apheresis using the ratio calculated in step b); and
d) collecting the plasma;

wherein the amount of AC in the collected plasma is less or equal to 9.1%.

in the method of the present invention, the HCT can be measured externally during donor screening, because HCT is a usual hematology parameter. HCT is the ratio (or percentage) of the volume of RBC to the volume of whole blood. in addition, HCT can be measured internally using the collection equipment during process. The HCT used to calculate required AC pump ratio can use either value, or some

combination, proportion or sequence of both external and internal values. HCT value accuracy can improve the degree of control of the process. Therefore, preferably said HCT value could be communicated directly to the collection equipment.

Also optionally, in the method of the present invention, the above steps (a) and (b) can be repeated during the apheresis process if desired, and therefore the ratio can vary during the apheresis process, especially when the patient's HCT is measured internally using the collection equipment.

The ratio of the AC flow pump to anticoagulated blood flow pump could be targeted to try and hit both FDA nomogram targets (donor volume and collected volume) as closely as possible, or some variable amount of compensation that diminishes the excess AC present on higher HCT collections, but still offers advantages over the current manifestations. The AC pump could be controlled at an alternate (variable) ratio to the anticoagulated blood pump, and/or incorporate the time rate of change observed in the collected volume in the optimal targeted AC flow calculation.

in the method of the present invention, the AC to anticoagulated blood ratio is less than 1:16; i.e., less than 0.06. Thus, it is possible to increase the anticoagulated blood amount or flow keeping constant the AC amount or flow. in a preferred embodiment, said ratio can range between 1:17 and 1:24, depending on the HCT value of the donor. in other preferred embodiments said ratio can be 1:17, 1:18, 1:19, 1:20, 1:21, 1:22, 1:23, or 1:24.

Preferably, the apheresis of the method of the present invention is a plasmapheresis, more preferably is a low-volume plasmapheresis. As used herein, the term "low-volume plasmapheresis" refers to plasma exchange treatments involving plasma collected volumes between 600 mL and 900 mL.

There is no a single unique correlation or curve between the AC flow and the HCT that must be used in the method of the present invention. The inventive concept is that the ratio can vary according to HCT of the donor, and not remain fixed for each donation. The optimal mode of targeting a certain ratio can be optimized according to a variety of factors. Said correlation can be obtained based on a balance benefit (of increased collection concentration with the reduction of excess AC) and hazards of the coagulation cascade commencing during the collection process where it is not intended, as well as other operational or risk factors.

The invention will be described below with reference to the figures, by way of explanatory, non-restrictive examples, of various embodiments of the present invention, in which:

Figure 1 shows the design of the plasmapheresis device according to the present invention.

Figure 2 shows graphics of AC to anticoagulated blood ratios, volume of plasma from donor (Vd), protein concentration in the collected plasma (Protein conc), and the amount of AC in collected volume relative to nomogram (AC/intent) as a function of the HCT value (X-axis).

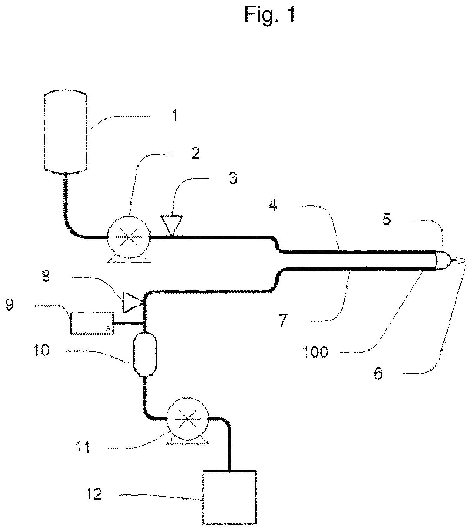

As shown in figure 1, as the collection is commenced Y connector 5 has been connected to needle 6 which is in a vein in the donor's arm. Currently available manifestations of collection equipment propel the flow of AC from pump 2 at a fixed ratio to the flow that is drawn from the donor drawn through pump 11. in the various currently available examples, this ratio is accomplished by either using similar tubing with different speed peristaltic pumps, or similar speed peristaltic pumps driving tubing with different diameters. For example, for a ratio of 1:17, one unit of flow from the AC pump 2, per 17 units of flow through pump 11. The portion of RBC in the flow stream drawn by pump 11 through line 7 and approaching separation device 12 will vary according to the donor's HCT.

**EXAMPLES**

Example 1: Evaluation of the content of AC in collected blood using the method of the present invention.

When these modified algorithm ratios from the previous example are modelled closer adherence to the FDA guidance intended volume is evident. Five instances are illustrated in table 1 for an 880 mL collection intent.

As a result of employing this alternate ratio, the collected volume is comprised of plasma and AC components that conform to the FDA guidance document for all instances of HCT.

Concentration of protein in the collected volume has increased, with less AC dilution. The FDA intended collection amounts can be achieved without bottles with excess fill that may be difficult to extract. The efficiency of fractionation will increase (more output for the same liquid volumetric input/effort). If a particular business is constrained by fractionation input rate- it may be possible that overall business output could increase.

Table 1 shows collection volume intent of 880 mL with different levels of HCT, between 38% to 54%, using different alternate pump flow ratios. The amount of anticoagulant in the collected plasma is consistent (9% v/v) for all levels of HCT. If this amount is sufficient to prevent coagulation, then in all cases of HCT there will not be excess applied that may have deleterious effects on proteins that are desired to be recovered in the process and later delivered to patients as medicine.

Table 1. Content of AC in collected blood.

| Collection volume intent (mL) | HCT of donor | Example Alternate Pump Flow Ratio | Resultant AC in Collected plasma (mL) | Total plasma collected (mL) |
|---|---|---|---|---|
| 880 | 38% | 17.12903 | 80.00 (9%) | 800.00 |
| 880 | 41% | 17.94915 | 80.00 (9%) | 800.00 |
| 880 | 44% | 18.85715 | 80.00 (9%) | 800.00 |
| 880 | 49% | 20.60785 | 80.00 (9%) | 800.00 |
| 880 | 54% | 22.73913 | 80.00 (9%) | 800.00 |

Example 2: Measurement of HCT using the collection equipment to infer the AC amount in the collected plasma.

When HCT measurement become increasing significant in the modified process (both donor screening, and now also control of collection equipment), it may be beneficial to increase the quality of this measurement. it may be possible to use the collection equipment itself to infer HCT from the physical characteristics of what is being drawn. This enhanced quality of HCT measurement gained from operation may be used to adjust the targeted AC ratio during the course of the collection.

As it is shown in Figure 1, an example of using the collection equipment to infer AC, would be to monitor the accumulation of overall flow through pump 11 required to fill the separation chamber 12 with RBC's to a detectable amount that is used to start the RBC return cycle. Since the geometry of the separation chamber is known "x" by softgoods equipment design- then by using the accumulated flow past pump 11 of RBC+Plasma+AC at the moment RBC's of volume "x" have collected- a calculation of HCT can be made.

Another example of inferring HCT on collection equipment is once the separation chamber and tubing to the collection container has been filled- additional flow through pump 11 will increase volume in the collection container. The collection container is on a load cell, and it may be possible to infer HCT by comparing the rate of flow through pump 11 (which is known and includes the RBCs+Plasma+AC) and the time rate of change of separated plasma entering the collection container (Plasma+AC).

Example 3: Different ratio algorithms depending of donor's HCT that can be used in the method of the present invention.

As it would be necessary to balance benefit (of increased collection concentration with the reduction of excess AC) and hazards of the coagulation cascade commencing during the collection process where it is not intended, or other operational factors that need to be included in seeking the optimal conditions.

Figure 2 shows some of the options for the formula ratio depending on the amount of the HCT. These or other approaches become possible once the constraint of a fixed ratio is removed. Line A) shows the previous example illustrating a theoretically preferential set of ratios that could result in a consistent amount of AC for all donor's HCT values. Line B) illustrates the comparison to a static ratio, without regard to HCT (current general practice is a fixed 1:16).

A variety of other approaches could be taken. Some (non-limiting) are illustrated in Line C) which is a less aggressive HCT to Ratio relationship (average of Line A and 16:1) and Line D) which is an option that puts a ceiling on the ratio (this example at 18.7).

As shown in Figure 2, using the method of the present invention it is also possible to obtain a higher amount of plasma from a donor (Vd) when the HCT value is higher than 44%, and also a higher protein concentration (Protein conc) and lower amount of AC in the collected volume relative to nomogram intent

(Ac/intent).

As can be seen in Figure 2.2 using different approaches following the method of the present invention (lines A, C, and D) it is possible to obtain a higher amount of plasma from a donor (Vd) as compared with the current practice (Ratio 1:16, line B) which falls short of collecting the intended amount. For example with a collection of an upper weight group donor with an HCT of 44, the volume drawn from the donor is intended to be 800mL, but ends up being only 786mL due to the excess AC in the collected volume. Across the illustrated range of HCT, the fixed 1:16 ratio drawn with line B shows the amount drawn from the donor varies from 795-767mL. in all cases- lower than the 800 mL intent.

in addition, as shown in Figure 2.3, it is also possible to get a higher protein concentration in the collected plasma using any of the approaches following the method of the present invention (lines A, C, and D) compared with the current practice (Ratio 1:16, line B). For example, with an HCT of 44- the protein concentration in the collected volume would be 98.3% of the value expected if the intended amount of AC were present. Across the illustrated range of HCT, the fixed 1:16 ratio drawn with line B shows this dilution effect varies from 99.3% to 96.1%.

The amount of AC relative to the 9% intent is illustrated in Figure 2.4 line A using this variable ratio approach. This is in comparison to line B (a fixed 1:16 ratio) where the AC in the collected material can be considerably higher than the intent.

## Claims

1. Method for controlling the concentration of an anticoagulant (AC) composition added to a donor's plasma during a fixed volume apheresis extraction process, the method comprising utilising a donor's hematocrit (HCT) measurement to determine the ratio of the anticoagulant composition to the donor's uncoagulated blood during the apheresis extraction process,

   wherein ratio of the anticoagulant composition to the donor's uncoagulated blood during the apheresis extraction process is 1:R, wherein R is calculated according to the following equation:

   $$R = \frac{V_C/V_{AC} - HCT}{1 - HCT}$$

   wherein $V_C$ is the total volume of collected plasma (mL); $V_{AC}$ is the volume of anticoagulant in the collected plasma (mL), and HCT is the patient's hematocrit expressed in decimals.

2. Method, according to claim 1, comprising the steps of:

   a) measuring the HCT value in said donor's blood;
   b) calculating the AC to anticoagulated blood ratio in view of said HCT value of step a);
   c) carrying out the apheresis using the ratio calculated in step b); and
   d) collecting the plasma.

   wherein the amount of AC in the collected plasma is less or equal to 9.1% (v/v).

3. Method, according to claim 1, wherein said HCT value is measured externally during donor screening prior to collection.

4. Method, according to claim 1, wherein said HCT value is measured internally using the apheresis device during process.

5. Method, according to any of the preceding claims, wherein said HCT value is a combination of an external value and an internal value.

6. Method, according to claim 2, wherein said steps (a) and (b) are repeated during apheresis process to recalculate said ratio R.

7. Method, according to any of the preceding claims, wherein the AC to anticoagulated blood ratio is less than 1:16.

8. Method, according to claim 7, wherein said ratio ranges between 1:17 and 1:24.

9. Method, according to claim 7, wherein said ratio is 1:17, 1:18, 1:19; 1:20; 1:21; 1:22; 1:23; or 1:24.

10. Method, according to any of the preceding claims, wherein said apheresis is a plasmapheresis.

11. Method, according to claim 10, wherein said plasmapheresis is a low-volume plasmapheresis, involving plasma collected volumes between 600 mL and 900 mL.

**Patentansprüche**

1. Verfahren zum Steuern der Konzentration einer gerinnungshemmenden Zusammensetzung (AC), die dem Plasma eines Spenders während eines Apherese-Extraktionsverfahrens mit festem Volumen zugesetzt wird, wobei das Verfahren die Verwendung der Messung des Hämatokrits (HCT) eines Spenders umfasst, um das Verhältnis der gerinnungshemmenden Zusammensetzung zu dem nicht geronnenen Blut des Spenders während des Apherese-Extraktionsverfahrens zu bestimmen,
wobei das Verhältnis der gerinnungshemmenden Zusammensetzung zu dem unkoagulierten Blut des Spenders während des Apherese-Extraktionsverfahrens 1:R beträgt, wobei R gemäß der folgenden Gleichung berechnet wird:

$$R = \frac{V_C/V_{AC} - HCT}{1 - HCT}$$

wobei VC das Gesamtvolumen des gesammelten Plasmas (mL), VAC der Anteil des Antikoagulans im gesammelten Plasma (mL) und HCT der in Dezimalzahlen ausgedrückte Hämatokrit des Patienten ist.

2. Verfahren nach Anspruch 1, das die folgenden Schritte umfasst::

   a) Messen des HCT-Wertes im Blut des Spenders;
   b) Berechnen des Verhältnisses von AC zu antikoaguliertem Blut unter Berücksichtigung des HCT-Wertes aus Schritt a);
   c) Durchführen der Apherese unter Verwendung des in Schritt b) berechneten Verhältnisses; und
   d) Sammeln des Plasmas.

   wobei die Menge an AC in dem gesammelten Plasma weniger oder gleich 9,1 % (v/v) beträgt.

3. Verfahren nach Anspruch 1, wobei der HCT-Wert während des Spenderscreenings vor der Entnahme extern gemessen wird.

4. Verfahren nach Anspruch 1, wobei der HCT-Wert intern unter Verwendung der Apheresevorrichtung während des Prozesses gemessen wird.

**5.** Verfahren nach einem der vorhergehenden Ansprüche, wobei der HCT-Wert eine Kombination aus einem externen Wert und einem internen Wert ist.

**6.** Verfahren nach Anspruch 2, wobei die Schritte (a) und (b) während des Aphereseprozesses wiederholt werden, um das Verhältnis R neu zu berechnen.

**7.** Verfahren nach einem der vorhergehenden Ansprüche, wobei das Verhältnis von AC zu antikoaguliertem Blut weniger als 1:16 beträgt.

**8.** Verfahren nach Anspruch 7, wobei das Verhältnis zwischen 1:17 und 1:24 liegt.

**9.** Verfahren nach Anspruch 7, wobei das Verhältnis 1:17, 1:18, 1:19; 1:20; 1:21; 1:22; 1:23; oder 1:24 beträgt.

**10.** Verfahren nach einem der vorhergehenden Ansprüche, wobei die Apherese eine Plasmapherese ist.

**11.** Verfahren nach Anspruch 10, wobei die Plasmapherese eine niedrigvolumige Plasmapherese ist, bei der Plasmasammelvolumina zwischen 600 mL und 900 mL verwendet werden.

**Revendications**

**1.** Procédé de régulation de la concentration d'une composition anticoagulante (AC) ajoutée au plasma d'un donneur au cours d'un processus d'extraction par aphérèse à volume fixe, le procédé comprenant l'utilisation d'une mesure de l"hématocrite d'un donneur (HCT) pour déterminer le rapport de la composition anticoagulante au sang non coagulé du donneur pendant le processus d'extraction par aphérèse,

dans lequel le rapport entre la composition anticoagulante et le sang non coagulé du donneur pendant le processus d'extraction par aphérèse est de 1:R, dans lequel R est calculé selon l'équation suivante :

$$R = \frac{V_C/V_{AC} - HCT}{1 - HCT}$$

dans lequel Vc est le volume total de plasma prélevé (mL) ; $V_{AC}$ est le volume d'anticoagulant dans le plasma prélevé (ml), et HCT est l'hématocrite du patient exprimé en décimales.

**2.** Procédé, selon la revendication 1, comprenant les étapes consistant à :

a) mesurer la valeur HCT dans ledit sang du donneur ;
b) calculer le rapport AC au sang anticoagulé en fonction de la valeur HCT de l'étape a) ;
c) effectuer l'aphérèse en utilisant le rapport calculé à l'étape b) ; et
d) prélever le plasma.

dans lequel la quantité d'AC dans le plasma prélevé est inférieure ou égale à 9,1 % (v/v).

**3.** Procédé selon la revendication 1, dans lequel ladite valeur HCT est mesurée de manière externe lors de la sélection du donneur avant le prélèvement.

**4.** Procédé selon la revendication 1, dans lequel ladite valeur HCT est mesurée en interne à l'aide du dispositif d'aphérèse pendant le processus.

**5.** Procédé selon l'une quelconque des revendications précédentes, dans lequel ladite valeur HCT est une combinaison d'une valeur externe et d'une valeur interne.

**6.** Procédé selon la revendication 2, dans lequel lesdites étapes (a) et (b) sont répétées pendant le processus d'aphérèse pour recalculer ledit rapport R.

7. Procédé selon l'une quelconque des revendications précédentes, dans lequel le rapport AC au sang anticoagulé est inférieur à 1:16.

8. Procédé selon la revendication 7, dans lequel ledit rapport est compris entre 1:17 et 1:24.

9. Procédé selon la revendication 7, dans lequel ledit rapport est de 1:17, 1:18, 1:19 ; 1:20; 1:21 ; 1:22 ; 1:23; ou 1:24.

10. Procédé selon l'une quelconque des revendications précédentes, dans lequel l'aphérèse est une plasmaphérèse.

11. Procédé, selon la revendication 10, dans lequel ladite plasmaphérèse est une plasmaphérèse de faible volume, impliquant des volumes de plasma prélevés compris entre 600 ml et 900 ml.

# Fig. 1

Figure 2

**EP 4 007 917 B1**

## REFERENCES CITED IN THE DESCRIPTION

### Patent documents cited in the description

- US 5387187 A
- US 5494592 A
- US 5637082 A
- WO 2009129140 A
- WO 1994012223 A1
- WO 2019085156 A1
- JP 2012081213 A
- EP 2896415 A1